Europäisches Patentamt

European Patent Office

Office européen des brevets

(19)

(11) Veröffentlichungsnummer: **0 400 293**
**A1**

(12) **EUROPÄISCHE PATENTANMELDUNG**

(21) Anmeldenummer: 90105724.0

(22) Anmeldetag: 26.03.90

(51) Int. Cl.5: **A61B 6/04, A61B 17/22**

(30) Priorität: 01.06.89 DE 3917858

(43) Veröffentlichungstag der Anmeldung:
05.12.90 Patentblatt 90/49

(84) Benannte Vertragsstaaten:
CH ES FR GB IT LI

(71) Anmelder: **DORNIER MEDIZINTECHNIK GMBH**
**Postfach 1128**
**D-8034 Germering 1(DE)**

(72) Erfinder: **Viebach, Thomas, Dipl.-Ing.**
**Ammerhof 2**
**D-8121 Pähl(DE)**

(74) Vertreter: Landsmann, Ralf, Dipl.-Ing.
c/o DORNIER GMBH Postfach 1420
D-7990 Friedrichshafen 1(DE)

(54) Koppelfläche für einen Lithotripter.

(57) Patientenliege für die Behandlung mit extrakorporal erzeugten Stosswellen, insbesondere für einen Lithotripter, mit einer Liegefläche (L) und einer Koppelfläche (K), durch welche die Stosswellen in den Patientenkörper (PK) eintreten. Zur Vermeidung der unbeabsichtigten Patientenverschiebung ist die Koppelfläche (K) steif (starr) ausgebildet.

# Fig. 1

EP 0 400 293 A1

## Koppelfläche für einen Lithotripter

Die Erfindung betrifft eine Patientenliege für die Behandlung mit extrakorporal erzeugten Stosswellen, insbesondere für einen Lithotripter nach dem Oberbegriff des Anspruchs 1.

Bei Lithotriptoren wurde bisher die Stosswellenankopplung über ein offenes Wasserbad oder ein mit einer flexiblen Membran abgeschlossenes Wasserkissen realisiert. Die Vorteile des geschlossenen Wasserkissens in der klinischen Praxis sind unumstritten, jedoch ergeben sich auch Nachteile, wie zum Beispiel die aufwendige Druckregelung, eine unbeabsichtigte Patientenverschiebung beim Ankoppeln und Positionieren, eine Faltenbildung des Kissens oder Kollisionsprobleme.

Aus der DE-PS 35 32 678 ist eine Liege mit einem Fenster mit Nasser gefüllter Vorlaufstrecke mit oder ohne Membranabdeckung bekannt. Auch dabei kann eine Druckänderung im Wasser zu einer unbeabsichtigten Patientenverschiebung führen.

Aufgabe der Erfindung ist es, eine Patientenliege vorzuschlagen, bei der eine unbeabsichtigte Patientenberührung oder -verschiebung beim Positionieren nicht erfolgt.

Diese Aufgabe wird erfindungsgemäss gelöst von einer Patientenliege mit den Merkmalen des Anspruchs 1. Ausführungen der Erfindung sind Gegenstände von Unteransprüchen.

Die Erfindung beruht darauf, die Koppelfläche zwischen dem Patienten und der Stosswellenquelle oder dem Ankoppelmedium steif auszubilden. Steif im Sinne der Erfindung ist eine Fläche, die beim Auflegen des Patienten nicht oder nur unwesentlich ihre Form verändert (formstabil, starr).

Die Form der Patientenliege ist im wesentlichen in Längsrichtung eben und quer dazu konkav (also einer Rinne entsprechend).

In einer anderen Ausführung kann der Bereich der Ankoppelfläche in Patientenlängsrichtung konvex und in Patientenquerrichtung konkav sein, das heisst er kann sattelförmig ausgebildet sein. Bei kleineren Ankoppelflächen sind auch ganz ebene oder in beiden Richtungen konvex gekrümmte Geometrien möglich.

Die Stosswelleneinheit befindet sich regelmässig unter diesem Koppelfenster, zum Beispiel in einem Wasserbad.

Folgende Vorteile ergeben sich durch die Erfindung:
- Der Patient liegt bequem, ohne die Gefahr des Durchfallens und ohne drückende Liegenkanten.
- Der Patient spürt absolut nichts von den beim Positionieren auftretenden Relativbewegungen zwischen ihm un der Stosswelleneinheit.
- Beim Positionieren treten keine Kräfte auf, die den Patienten und den Stein verlagern könnten.
- Die Stosswelleneinheit kann jederzeit (zum Beispiel für die Röntgenortung) weggefahren und anschliessend an den Ausgangspunkt zurückgefahren werden, ohne die eingestellte Steinlage durch An- und Abkoppeln zu verändern.
- Für die Patienten besteht keinerlei Gefährdung durch Kollisionen mit der Stosswelleneinheit oder mit einem dort befestigten Ultraschall- oder Röntgeneinrichtungen.
- Eine genaue Druckregelung ist überflüssig.

Die Ausbildung des Ankoppelfensters ist so, dass alle Patienten darauf über längere Zeit liegen können und gleichzeitig ein grossflächiger Hautkontakt gewährleistet ist. Der Körperkontakt kann ausser durch Koppelgel durch Zuhilfenahme von gallertähnlichen Massen (zum Beispiel sogenannten Ultraschallvorlaufstrecken wie Mitteln, die unter den Bezeichnungen: Sonar-aid oder Reston im Handel sind) verbessert werden.

Auswahlkriterien für das Material sind unter anderem die akustische Impedanz, die Röntgendurchlässigkeit, die mechanische Steifigkeit und die Verarbeitbarkeit. Polyäthylen ist in diesen vier Punkten gut geeignet.

Die Stosswellenquelle kann sich entweder in einem Wasserbad unter dem Fenster bewegen oder sie ist beweglich in einem Topf aufgehängt, dessen oberer Rand am Fenster abdichtet. Dieser Topf ist dann unter dem Fenster verschiebbar aufgehängt.

Die Erfindung wird anhand von drei Figuren näher beschrieben.

Es zeigen:

Figur 1 eine erfindungsgemässe Liege.

Figur 2 zwei Möglichkeiten der Ankoppelung des Patienten.

Figur 3 zwei Möglichkeiten der Anbringung einer Stosswellenquelle.

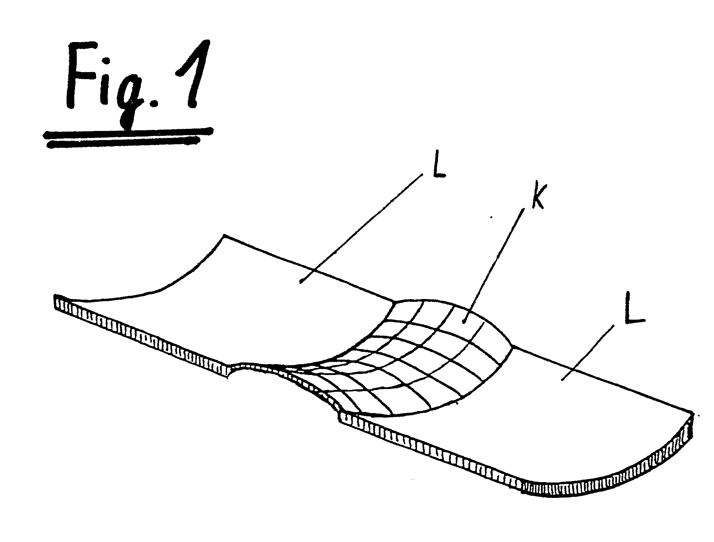

Figur 1 zeigt eine Patientenliege, bestehend aus den Liegeflächen L und der Koppelfläche K. Die Koppelfläche K ist hier dreidimmensional gekrümmt, sattelförmig ausgebildet. Die Krümmung in Patientenlängsrichtung ist konvex, in Patientenquerrichtung konkav. Die Liegeflächen L sind ebenfalls konkav gekrümmt.

Die Koppelfläche kann zum Beispiel durch Tiefziehen einer Thermoplastplatte hergestellt sein.

Figur 2 zeigt links einen Patientenkörper PK auf der Koppelfläche K in Rückenlage (Wirbelsäule und Nieren eingezeichnet). Da die Koppelfläche K erfindungsgemäss starr ist, sind Flächen vorhanden, an denen sich der Patientenkörper PK nicht an die Koppelfläche K anschmiegt. Diese Flächen sind hier mit einem Koppelgel (dunkel gezeichnet) gefüllt. Figur 2 zeigt rechts einen Patientenkörper PK in Bauchlage auf der Koppelfläche K. Da sich der Bauch des Patienten besser an die Koppelfläche K anschmiegt, ist hier weniger Koppelgel erforderlich.

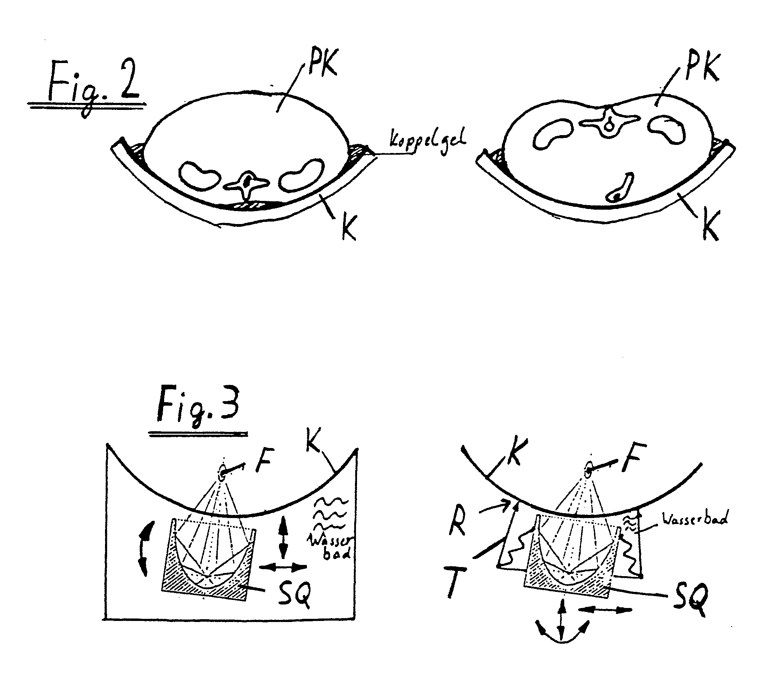

Figur 3 zeigt zwei Varianten der Anbindung einer Stosswellenquelle SQ an die Koppelfläche K. Links befindet sich die fokussierende Stosswellenquelle SQ in einem Wasserbad unter der Koppelfläche. Eingezeichnet ist die Stosswellenquelle SQ mit ihrem Brennpunkt F, in dem sich das zu zerstörende Konkrement befindet. Die Stosswellenquelle besitzt zur Positionierung mehrere Freiheitsgrade der Bewegung und Rotation, wie eingezeichnet.
In Figur 3 ist rechts eine weitere Ausführung gezeigt, bei der die Stosswellenquelle SQ in einem Topf T unter der Koppelfläche K aufgehangen ist. Das Wasserbad zur Ankopplung wird dadurch wesentlich kleiner. Der obere Rand des Topfes T dichtet an der Koppelfläche K ab. Der Topf kann verschiebbar sein.

## Ansprüche

1. Patientenliege für die Behandlung mit extrakorporal erzeugten Stosswellen, insbesondere für einen Lithotripter, mit einer Liegefläche (L) und einer Koppelfläche (K), durch welche die Stosswellen in den Patientenkörper (PK) eintreten, **dadurch gekennzeichnet**, dass die Koppelfläche (K) steif ist.

2. Patientenliege nach Anspruch 1, dadurch gekennzeichnet, dass die Koppelfläche (K) in Patientenlängsrichtung eben und quer dazu konkav ist.

3. Patientenliege nach Anspruch 1, dadurch gekennzeichnet, dass die Koppelfläche (K) in Patientenlängsrichtung konvex und quer dazu konkav ist.

4. Patientenliege nach Anspruch 1, dadurch gekennzeichnet, dass die Koppelfläche (K) in beiden Richtungen eben oder konvex ist.

5. Patientenliege nach einem der vorhergehenden Ansprüche, dadurch gekennzeichnet, dass die Koppelfläche (K) aus Polyäthylen besteht und eine im wesentlichen gleichmässige Dicke aufweist.

6. Patientenliege nach einem der vorhergehenden Ansprüche, dadurch gekennzeichnet, dass die Koppelfläche (K) aus einem Kunststoff mit einer ähnlichen akustischen Impedanz besteht wie Wasser.

Fig. 1

Fig. 2

PK

koppelgel

K

PK

K

Fig. 3

F

K

Wasser bad

SQ

K

F

R

T

Wasserbad

SQ

Europäisches
Patentamt

# EUROPÄISCHER RECHERCHENBERICHT

Nummer der Anmeldung

EP 90 10 5724

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 294 679 (SIEMENS AG BERLIN UND MUENCHEN) <br> * Anspruch 1; Figur 1 * <br> --- | 1 | A 61 B 6/04 <br> A 61 B 17/22 |
| A | DE-C-3 532 678 (RICHARD WOLF GMBH) <br> * Anspruch 1; Figur 1 * <br> ----- | 1 | |

RECHERCHIERTE SACHGEBIETE (Int. Cl.5)

A 61 B

Der vorliegende Recherchenbericht wurde für alle Patentansprüche erstellt

| Recherchenort | Abschlußdatum der Recherche | Prüfer |
|---|---|---|
| BERLIN | 30-08-1990 | PAPA E.R. |